# FASCICULE DE BREVET EUROPEEN

(11) **EP 2 900 077 B1**
(45) Date de publication et mention de la délivrance du brevet: **12.02.2020**
(21) Numéro de dépôt: 13730003.4
(22) Date de dépôt: 29.05.2013
(51) Int. Cl.: A61K 39/07, A61K 39/09, A61K 39/02, A23K 10/18, A61K 39/00, A23K 50/00, A23K 50/75

(54) **COMPOSITION BACTÉRIENNE DESTINÉE AU TRAITEMENT DE LA COLIBACILLOSE DANS LES ÉLEVAGES EN PARTICULIER LES ÉLEVAGES AVIAIRES AINSI QU'EAU DE BOISSON RENFERMANT UNE TELLE COMPOSITION BACTÉRIENNE**
BAKTERIELLE ZUSAMMENSETZUNG ZUR BEHANDLUNG VON COLIBACILLOSE AUF FARMEN, INSBESONDERE GEFLÜGELFARMEN, UND VON TRINKWASSER MIT SOLCH EINER BAKTERIELLEN ZUSAMMENSETZUNG
BACTERIAL COMPOSITION FOR THE TREATMENT OF COLIBACILLOSIS ON FARMS, IN PARTICULAR POULTRY FARMS, AND ALSO DRINKING WATER CONTAINING SUCH A BACTERIAL COMPOSITION

(30) Priorité: 30.05.2012 FR 1254976
(43) Date de publication de la demande: 05.08.2015
(73) Titulaire: Nolivade, 53810 Change (FR)
(72) Inventeur: DE BRUEKER, Marc, F-53810 Changé (FR); THEBAULT, Stéphane, F-53240 Germain le Fouilloux (FR)
(74) Mandataire: Monni, Richard
(86) Numéro de dépôt international: PCT/FR2013/051202
(87) Numéro de publication internationale: WO 2013/178947

(56) Documents cités:
- WO-A1-92/12639
- KR-A- 20110 125 810
- US-A1- 2002 146 399
- US-A1- 2011 287 157
- MANEEWAN C., YAMAUCHI K., THIRABUNYANON M., SIRI S., MEKBUNGWAN A. AND THONGWITTAYA N.: "Development of Bacillus subtilis MP and effective utilization on productivity and microorganisms in feces of suckling piglets", INTERN. J. APPL. RES. VET. MED., vol. 9, no. 4, 2011, pages 382-387, XP002688379,
- KYRIAKIS S C ET AL: "THE EFFECT OF PROBIOTIC LSP 122 ON THE CONTROL OF POST-WEANING DIARRHOEA SYNDROME OF PIGLETS", RESEARCH IN VETERINARY SCIENCE, BRITISH VETERINARY ASSOCIATION, LONDON, GB, vol. 67, no. 3, 1 janvier 1999 (1999-01-01), pages 223-228, XP008003110, ISSN: 0034-5288, DOI: 10.1053/RVSC.1999.0308
- TEO A Y-L ET AL: "Inhibition of Clostridium perfringens by a novel strain of Bacillus subtilis isolated from the gastrointestinal tracts of healthy chickens", APPLIED AND ENVIRONMENTAL MICROBIOLOGY, AMERICAN SOCIETY FOR MICROBIOLOGY, US, vol. 71, no. 8, 1 août 2005 (2005-08-01), pages 4185-4190, XP002375508, ISSN: 0099-2240, DOI: 10.1128/AEM.71.8.4185-4190.2005

## Description

La présente invention a pour objet une composition bactérienne destinée au traitement de la colibacillose dans les élevages d'animaux en particulier les élevages aviaires.

La volaille constitue la seconde viande la plus consommée dans le monde avec de plus 91 millions de tonnes en 2009.

La production française s'élève à environ 1 650 000 Tec (tonne équivalent carcasse) dont 50 % de poulet, ce qui est considérable.

L'infection par la bactérie Escherichia coli qui représente une très large proportion des infections bactériennes chez la volaille constitue un véritable fléau pour les éleveurs aviaires et peut entrainer des pertes économiques très importantes.

Chez l'oiseau, la bactérie Escherichia coli correspond en règle générale à un agent contaminant opportuniste qui créé des infections en présence de divers facteurs contaminants : mode d'élevage intensif, conditions d'élevage (insuffisance au niveau de la gestion de la qualité de l'eau, de la ventilation, du chauffage...), présence de virus ou de diverses bactéries pathogènes...

Ces facteurs contaminants entrainent un affaiblissement et une immunodépression des animaux de nature à permettre à E.coli de les infecter avec toutes les conséquences néfastes qui en résultent.

Pour lutter contre ce fléau, les éleveurs aviaires s'efforcent de prendre un maximum de mesures préventives systématiques pour garantir dans leurs élevages des conditions d'hygiène strictes de nature à empêcher autant que possible le développement de germes pathogènes ; à cet effet, ils effectuent en particulier des opérations de nettoyage et de désinfection régulières de leurs locaux, notamment pendant les périodes de vide sanitaire.

Un autre impératif essentiel pour combattre le développement de germes pathogènes consiste à faire en sorte que l'eau de boisson des volailles ait toujours une qualité irréprochable, même et surtout dans les abreuvoirs.

Parallèlement à ces traitements préventifs, le seul traitement curatif actuellement proposé aux éleveurs aviaires pour traiter une colibacillose déjà déclarée est l'antibiothérapie qui présente de nombreux inconvénients.

En premier lieu, un tel traitement est de plus en plus difficile à prescrire du fait de l'abattage précoce des animaux, des délais d'attente importants...

Toutefois, le principal inconvénient d'un traitement par antibiotiques en médecine vétérinaire est lié aux phénomènes croissants d'antibio-resistance ; par suite, pour ne pas favoriser l'émergence de germes multi-résistants aux antibiotiques, les autorités sanitaires sont actuellement très vigilantes sur l'usage des antibiotiques en élevage, et pour cette raison, l'usage de certains antibiotiques pourrait même à plus ou moins long terme être limité voire interdit.

Il serait donc souhaitable de pouvoir proposer aux éleveurs aviaires un moyen de traitement de la colibacillose déjà déclarée pouvant constituer une alternative à l'antibiothérapie.

L'invention a pour objet de proposer une composition de nature à satisfaire à cette demande.

Dans le contexte susmentionné, il a été constaté conformément à l'invention que des éleveurs et en particulier des éleveurs aviaires utilisent différentes compositions bactériennes, renfermant en particulier des bactéries du genre bacillus pour nettoyer et désinfecter leurs locaux, notamment pendant les périodes de vide sanitaire.

Il a également été établi que des bactéries peuvent agir comme probiotiques en occupant l'espace dans le tube digestif des animaux de façon à y empêcher le développement de bactéries pathogènes telles que E.coli.

Or, on a imaginé que certaines souches de bactéries notamment de bactéries du genre bacillus pourraient également avoir, outre leurs propriétés probiotiques, des propriétés curatives pour le traitement de la colibacillose déjà installée, et on a effectué des recherches empiriques pour tenter d'isoler de telles souches.

On connaît notamment de l'état de la technique
- la demande WO1992012639 qui décrit l'utilisation de lactobacillus pour protéger le tractus intestinal des animaux, et en particulier des poules, contre les bactéries pathogènes,
- l'article de Maneewan et al. Intern. J. Appl. Res. Vet. 2011, Med, vol 9, no 4, pages 382 à 387, qui décrit deux souches de bactéries du genre Bacillus utilisées dans la cadre du traitement des diarrhées des porcs nouveaux-nés,
- la demande US20020146399 qui décrit l'utilisation de différentes bactéries en combinaison avec de l'acide sorbique en tant que probiotique animal,
- l'article de Kyriakis et al, Reasearch in Veterinary Sciences, British Veterinary Association, London GB, 1999, vol 67, no 3, pages 223 à 228 qui décrit un probiotique comprenant des spores viables de Bacillus licheniformis. et qui est utilisé dans le cadre des diarrhées du porcelet causées par des souches Escherichia coli entérotoxigéniques,
- ou encore la demande KR20110125810A qui décrit une composition probiotique issue de l'écorce interne séchée des pousses Cinnamomum verum, comprenant au moins un bacillus, un pediococcis ou un lactobacillus, pour améliorer la santé des animaux et en particulier la lutte contre les bactéries pathogènes intestinales.

Il a ainsi pu être établi conformément à l'invention que, de façon très surprenante, des souches particulières de Bacillus subtilis ou de Bacillus licheniformis peuvent agir en synergie avec certaines souches de bactéries lactiques pour inhiber le développement des colibacilles chez la volaille.

On peut décrire par example une composition bactérienne destinée au traitement de la colibacillose dans les éle-vages d'animaux en particulier les élevages aviaires caractérisée en ce qu'elle renferme d'une part au moins 10⁵ ufc/ml d'au moins l'une des quatre souches suivantes de Bacillus subtilis ou de Bacillus licheniformis : NOL 01, NOL 02, NOL 03 et NOL 04 et d'autre part au moins 10⁵ ufc/ml d'au moins une bactérie lactique choisie parmi les bactéries lactiques suivantes : Lactococcus lactis spp lactis 1 souche NOL 11 et Pediococcus pentosaceus 2 souche NOL 12.

Aussi, l'invention concerne-t-elle une composition bactérienne pour son utilisation dans le cadre du traitement de la colibacillose dans les élevages d'animaux en particulier les élevages aviaires, caractérisée en ce qu'elle renferme
- d'une part au moins 10⁵ ufc/ml d'au moins l'une des souches suivantes de Bacillus subtilis : NOL01 et NOL03,
- et d'autre part au moins 10⁵ ufc/ml d'au moins la bactérie lactique Lactococcus lactis spp lactis,
   où lesdites souches NOL01, NOL03 et NOL11 sont les suivantes :
   ∘ la souche NOL 01 est la souche déposée à l'Institut Pasteur sous le numéro CNCM I - 4606,
   ∘ la souche NOL 03 est la souche déposée à l'Institut Pasteur sous le numéro CNCM I - 4607, et
   ∘ la souche NOL 11 est la souche déposée à l'Institut Pasteur sous le numéro CNCM I - 4609.

Avantageusement, l'invention concerne la composition susmentionnée pour son utilisation décrite ci-dessus, caractérisée en ce qu'elle renferme entre 10⁵ et 10⁹ ufc/ml de bacillus et entre 10⁹ et 10¹⁰ ufc/ml de bactéries lactiques.

De manière avantageuse, l'invention concerne la composition susmentionnée pour son utilisation décrite ci-dessus, où lesdits bacillus et lesdites bactéries lactiques, sont sous forme sporulées et/ou végétatives.

De manière avantageuse, l'invention concerne la composition susmentionnée pour son utilisation décrite ci-dessus, caractérisée en ce qu'elle comprend l'une quelconque des compositions suivantes :
- la souche NOL 01 et la souche NOL 11, et
- la souche NOL 03 et la souche NOL 11.

L'invention concerne également une eau de boisson pour animaux d'élevage, en particulier pour volailles, caractérisée en ce qu'elle renferme la composition bactérienne susmentionnée.

En outre, l'invention concerne une composition bactérienne caractérisée en ce qu'elle renferme
- d'une part au moins 10⁵ ufc/ml d'au moins l'une des souches suivantes de Bacillus subtilis : NOL01 et NOL03,
- et d'autre part au moins 10⁵ ufc/ml d'au moins la bactérie lactique Lactococcus lactis spp lactis 1 souche NOL11,
   où lesdites souches NOL01, NOL03 et NOL11 sont les suivantes :
   ∘ la souche NOL 01 est la souche déposée à l'Institut Pasteur sous le numéro CNCM I - 4606,
   ∘ la souche NOL 03 est la souche déposée à l'Institut Pasteur sous le numéro CNCM I - 4607, et
   ∘ la souche NOL 11 est la souche déposée à l'Institut Pasteur sous le numéro CNCM I-4609.

De manière avantageuse, la composition susmentionnée caractérisée en ce qu'elle renferme entre 10⁵ et ufc/ml de bacillus et entre et 10¹⁰ ufc/ml de bactéries lactiques.

De manière avantageuse, la composition susmentionnée est telle qu'elle que lesdits bacillus et lesdites bactéries lactiques, sont sous forme sporulées et/ou végétatives.

De manière avantageuse, la composition susmentionnée est caractérisée en ce qu'elle comprend l'une quelconque des compositions suivantes :
- la souche NOL 01 et la souche NOL 11, et
- la souche NOL 03 et la souche NOL 11.

La composition conforme à l'invention peut avantageusement renfermer entre 10⁵ et 10⁹ ufc/ml de bacillus et entre 10⁹ et 10¹⁰ ufc/ml de bactéries lactiques.

Cette composition est destinée à être ajoutée à l'eau de boisson des animaux d'élevage en particulier des volailles.

L'invention concerne ainsi également une eau de boisson pour animaux d'élevage en particulier pour volaille caractérisée en ce qu'elle renferme la composition bactérienne susmentionnée.

A titre d'exemple, cette eau de boisson peut renfermer cette composition bactérienne en quantité de 10 à 100 ml dans 1000 l.

La composition qui fait l'objet de l'invention peut être proposée sous la forme de doses renfermant des bactéries sous forme sporulée et/ou végétative et se présentant sous une forme quelconque notamment des doses congelées et/ou ayant subi un traitement de lyophilisation ou d'atomisation.

On a, en effet, pu établir que dans une composition se présentant avec des bacillus sous la forme sporulée, une proportion importante de spores peuvent être remises à l'état végétatif, et donc redevenir actives dans le tube digestif de la volaille.

Les souches bactériennes pouvant être renfermées dans la composition qui fait l'objet de l'invention ont été identifiées par leur morphologie ainsi que par leurs caractéristiques biologiques et biochimiques.

Les résultats de cette identification sont mentionnés ci-dessous.

### 1/ Souche : NOL 01

| | |
|---|---|
| Genre et espèce : | Bacillus subtilis ou licheniformis |
| Morphologie : | long bacille gram + en chaînette |
| Caractères : | Milieu de culture : basique (PCA) ou gélose au sang |
| | Température : 30-37°C |
| | Aspect des colonies sur gélose au sang et PCA : |
| | Colonies jeunes et isolées : bombées, brillantes |
| Test de l'Oxydase + | |
| Test de la Catalase + | |

**Profil API 50CHB :**

| | Test | Résultat | | Test | Résultat |
|---|---|---|---|---|---|
| 1 | Glycérol | + | 26 | Salicine | + |
| 2 | Erythritol | - | 27 | D-Cellobiose | + |
| 3 | D-Arabinose | - | 28 | D-Maltose | + |
| 4 | L-Arabinose | + | 29 | D-Lactose | - |
| 5 | D-Ribose | + | 30 | D-Melibiose | + |
| 6 | D-Xylose | + | 31 | D-Saccharose | + |
| 7 | L-Xylose | - | 32 | D-Trehalose | + |
| 8 | D-Adonitol | - | 33 | Inuline | + |
| 9 | Méthyl-βD-Xylopyranoside | - | 34 | D-Mélézitose | - |
| 10 | D-Galacyose | - | 35 | D-Raffinose | + |
| 11 | D-Glucose | + | 36 | Amidon | + |
| 12 | D-Fructose | + | 37 | Glycogène | + |
| 13 | D-Mannose | + | 38 | Xylitol | - |
| 14 | L-Sorbose | - | 39 | Gentiobiose | - |
| 15 | L-Rhamnose | - | 40 | D-Turanose | - |
| 16 | Dulcitol | - | 41 | D-Lyxose | - |
| 17 | Inositol | + | 42 | D-Tagatose | - |
| 18 | D-Mannitol | + | 43 | D-Fucose | - |
| 19 | D-Sorbitol | + | 44 | L-Fucose | - |
| 20 | Méthyl-αD-Mannopyranoside | - | 45 | D-Arabitol | - |
| 21 | Méthyl-αD-Glucopyranoside | + | 46 | L-Arabitol | - |
| 22 | N-AcéthylGlucosamine | - | 47 | Potassium Gluconate | - |
| 23 | Amygdaline | + | 48 | Potassium 2-Cétogluconate | - |
| 24 | Arbutine | + | 49 | Potassium 5-Cétogluconate | - |
| 25 | Esculine citrate de fer | + | | | |

### 2/ Souche : NOL 02

| | |
|---|---|
| Genre et espèce : | Bacillus subtilis |
| Morphologie : | bacille gram + isolé ou par deux. Présence de spores. |
| Caractères : | Milieu de culture : basique (PCA) ou gélose au sang |
| | Température : 30-37°C |
| | Aspect des colonies sur gélose PCA: |
| | grande, plate, ronde, sèche, ancrée dans la gélose |
| | Aspect des colonies sur gélose au sang : |
| | non hémolytique, contour farineux |
| Test de l'Oxydase + | |
| Test de la Catalase + | |

**Profil API 50CHB :**

| | Test | Résultat | | Test | Résultat |
|---|---|---|---|---|---|
| 1 | Glycérol | + | 26 | Salicine | + |
| 2 | Erythritol | - | 27 | D-Cellobiose | + |
| 3 | D-Arabinose | - | 28 | D-Maltose | + |
| 4 | L-Arabinose | + | 29 | D-Lactose | - |
| 5 | D-Ribose | + | 30 | D-Melibiose | + |
| 6 | D-Xylose | - | 31 | D-Saccharose | + |
| 7 | L-Xylose | - | 32 | D-Trehalose | + |
| 8 | D-Adonitol | - | 33 | Inuline | + |
| 9 | Méthyl-βD-Xylopyranoside | - | 34 | D-Mélézitose | - |
| 10 | D-Galacyose | - | 35 | D-Raffinose | + |
| 11 | D-Glucose | + | 36 | Amidon | + |
| 12 | D-Fructose | + | 37 | Glycogène | + |
| 13 | D-Mannose | + | 38 | Xylitol | - |
| 14 | L-Sorbose | - | 39 | Gentiobiose | - |
| 15 | L-Rhamnose | - | 40 | D-Turanose | + |
| 16 | Dulcitol | - | 41 | D-Lyxose | - |
| 17 | Inositol | + | 42 | D-Tagatose | - |
| 18 | D-Mannitol | + | 43 | D-Fucose | - |
| 19 | D-Sorbitol | + | 44 | L-Fucose | - |
| 20 | Méthyl-αD-Mannopyranoside | - | 45 | D-Arabitol | - |
| 21 | Méthyl-αD-Glucopyranoside | + | 46 | L-Arabitol | - |
| 22 | N-AcéthylGlucosamine | - | 47 | Potassium Gluconate | - |
| 23 | Amygdaline | - | 48 | Potassium 2-Cétogluconate | - |
| 24 | Arbutine | + | 49 | Potassium 5-Cétogluconate | - |
| 25 | Esculine citrate de fer | + | | | |

### 3/ Souche : NOL 03

| | |
|---|---|
| Genre et espèce : | Bacillus subtilis |
| Morphologie : | bacille gram + isolé ou par deux. Présence de spores au gram. |
| Caractères : | Milieu de culture : basique (PCA) ou gélose au sang |
| | Température : 30-37°C |
| | Aspect des colonies sur gélose PCA : |
| | petite, plate, bord irrégulier, centre plus foncé |
| | Aspect des colonies sur gélose au sang : |
| | non hémolytique, centre légèrement creusé, bord net |
| Test de l'Oxydase + | |
| Test de la Catalase + | |

**Profil API 50CHB :**

| | Test | Résultat | | Test | Résultat |
|---|---|---|---|---|---|
| 1 | Glycérol | + | 26 | Salicine | + |
| 2 | Erythritol | - | 27 | D-Cellobiose | + |
| 3 | D-Arabinose | - | 28 | D-Maltose | + |
| 4 | L-Arabinose | + | 29 | D-Lactose | - |
| 5 | D-Ribose | - | 30 | D-Melibiose | + |
| 6 | D-Xylose | - | 31 | D-Saccharose | + |
| 7 | L-Xylose | - | 32 | D-Trehalose | + |
| 8 | D-Adonitol | - | 33 | Inuline | + |
| 9 | Méthyl-βD-Xylopyranoside | - | 34 | D-Mélézitose | - |
| 10 | D-Galacyose | - | 35 | D-Raffinose | + |
| 11 | D-Glucose | + | 36 | Amidon | + |
| 12 | D-Fructose | + | 37 | Glycogène | + |
| 13 | D-Mannose | + | 38 | Xylitol | - |
| 14 | L-Sorbose | - | 39 | Gentiobiose | - |
| 15 | L-Rhamnose | - | 40 | D-Turanose | + |
| 16 | Dulcitol | - | 41 | D-Lyxose | - |
| 17 | Inositol | + | 42 | D-Tagatose | - |
| 18 | D-Mannitol | + | 43 | D-Fucose | - |
| 19 | D-Sorbitol | + | 44 | L-Fucose | - |
| 20 | Méthyl-αD-Mannopyranoside | - | 45 | D-Arabitol | - |
| 21 | Méthyl-αD-Glucopyranoside | + | 46 | L-Arabitol | - |
| 22 | N-AcéthylGlucosamine | - | 47 | Potassium Gluconate | - |
| 23 | Amygdaline | + | 48 | Potassium 2-Cétogluconate | - |
| 24 | Arbutine | + | 49 | Potassium 5-Cétogluconate | - |
| 25 | Esculine citrate de fer | + | | | |

### 4/ Souche : NOL 04

| | |
|---|---|
| Genre et espèce : | Bacillus subtilis |
| Morphologie : | bacille gram + isolé ou par deux |
| Caractères : | Milieu de culture : basique (PCA) ou gélose au sang |
| | Température : 30-37°C |
| | Aspect des colonies sur gélose PCA : |
| | petite, plate, bord irrégulier, centre plus foncé |
| | Aspect des colonies sur gélose au sang : |
| | hémolytique, bord irrégulier |
| Test de l'Oxydase + | |
| Test de la Catalase + | |

**Profil API 50CHB :**

| | Test | Résultat | | Test | Résultat |
|---|---|---|---|---|---|
| 1 | Glycérol | + | 26 | Salicine | + |
| 2 | Erythritol | - | 27 | D-Cellobiose | + |
| 3 | D-Arabinose | - | 28 | D-Maltose | + |
| 4 | L-Arabinose | + | 29 | D-Lactose | - |
| 5 | D-Ribose | +/- | 30 | D-Melibiose | - |
| 6 | D-Xylose | - | 31 | D-Saccharose | + |
| 7 | L-Xylose | - | 32 | D-Trehalose | + |
| 8 | D-Adonitol | - | 33 | Inuline | +/- |
| 9 | Méthyl-βD-Xylopyranoside | - | 34 | D-Mélézitose | - |
| 10 | D-Galacyose | - | 35 | D-Raffinose | +/- |
| 11 | D-Glucose | + | 36 | Amidon | +/- |
| 12 | D-Fructose | + | 37 | Glycogène | +/- |
| 13 | D-Mannose | + | 38 | Xylitol | - |
| 14 | L-Sorbose | - | 39 | Gentiobiose | - |
| 15 | L-Rhamnose | - | 40 | D-Turanose | - |
| 16 | Dulcitol | - | 41 | D-Lyxose | - |
| 17 | Inositol | + | 42 | D-Tagatose | - |
| 18 | D-Mannitol | + | 43 | D-Fucose | - |
| 19 | D-Sorbitol | + | 44 | L-Fucose | - |
| 20 | Méthyl-αD-Mannopyranoside | - | 45 | D-Arabitol | - |
| 21 | Méthyl-αD-Glucopyranoside | +/- | 46 | L-Arabitol | - |
| 22 | N-AcéthylGlucosamine | - | 47 | Potassium Gluconate | - |
| 23 | Amygdaline | +/- | 48 | Potassium 2-Cétogluconate | - |
| 24 | Arbutine | +/- | 49 | Potassium 5-Cétogluconate | - |
| 25 | Esculine citrate de fer | + | | | |

### 5/ Souche : NOL 11

| | | |
|---|---|---|
| Genre et espèce : | Lactococcus lactis spp lactis 1 | |
| Morphologie : | coque gram + groupée par 2 en chaînettes | |
| Caractères : | Milieu de culture : MRS | |
| | Température : 30-37°C | |
| | Aspect des colonies sur | MRS : petites, brunes |
| | | Gélose au sang : petites, grises |
| Test de l'Oxydase - | | |
| Test de la Catalase - | | |

**Profil API 50CHL :**

| | Test | Résultat | | Test | Résultat |
|---|---|---|---|---|---|
| 1 | Glycérol | - | 26 | Salicine | + |
| 2 | Erythritol | - | 27 | D-Cellobiose | + |
| 3 | D-Arabinose | - | 28 | D-Maltose | + |
| 4 | L-Arabinose | - | 29 | D-Lactose | - |
| 5 | D-Ribose | + | 30 | D-Melibiose | - |
| 6 | D-Xylose | - | 31 | D-Saccharose | + |
| 7 | L-Xylose | - | 32 | D-Trehalose | + |
| 8 | D-Adonitol | - | 33 | Inuline | - |
| 9 | Méthyl-βD-Xylopyranoside | - | 34 | D-Mélézitose | - |
| 10 | D-Galacyose | + | 35 | D-Raffinose | - |
| 11 | D-Glucose | + | 36 | Amidon | + |
| 12 | D-Fructose | + | 37 | Glycogène | - |
| 13 | D-Mannose | + | 38 | Xylitol | - |
| 14 | L-Sorbose | - | 39 | Gentiobiose | - |
| 15 | L-Rhamnose | - | 40 | D-Turanose | - |
| 16 | Dulcitol | - | 41 | D-Lyxose | - |
| 17 | Inositol | - | 42 | D-Tagatose | - |
| 18 | D-Mannitol | + | 43 | D-Fucose | - |
| 19 | D-Sorbitol | - | 44 | L-Fucose | - |
| 20 | Méthyl-αD-Mannopyranoside | - | 45 | D-Arabitol | - |
| 21 | Méthyl-αD-Glucopyranoside | - | 46 | L-Arabitol | - |
| 22 | N-AcéthylGlucosamine | + | 47 | Potassium Gluconate | - |
| 23 | Amygdaline | - | 48 | Potassium 2-Cétogluconate | - |
| 24 | Arbutine | - | 49 | Potassium 5-Cétogluconate | - |
| 25 | Esculine citrate de fer | + | | | |

### 6/ Souche : NOL 12

| | | |
|---|---|---|
| Genre et espèce : | Pediococcus pentosaceus 2 | |
| Morphologie : | coque gram + groupée en paire ou en tétrade | |
| Caractères : | Milieu de culture : MRS | |
| | Température : 30-37°C | |
| | Aspect des colonies sur | MRS : blanches, laiteuses |
| | | Gélose au sang : petites, grises |
| Test de l'Oxydase - | | |
| Test de la Catalase - | | |

**Profil API 50CHL :**

| | Test | Résultat | | Test | Résultat |
|---|---|---|---|---|---|
| 1 | Glycérol | - | 26 | Salicine | + |
| 2 | Erythritol | - | 27 | D-Cellobiose | + |
| 3 | D-Arabinose | - | 28 | D-Maltose | + |
| 4 | L-Arabinose | + | 29 | D-Lactose | - |
| 5 | D-Ribose | + | 30 | D-Melibiose | + |
| 6 | D-Xylose | + | 31 | D-Saccharose | + |
| 7 | L-Xylose | - | 32 | D-Trehalose | + |
| 8 | D-Adonitol | - | 33 | Inuline | - |
| 9 | Méthyl-βD-Xylopyranoside | - | 34 | D-Mélézitose | - |
| 10 | D-Galacyose | + | 35 | D-Raffinose | + |
| 11 | D-Glucose | + | 36 | Amidon | - |
| 12 | D-Fructose | + | 37 | Glycogène | - |
| 13 | D-Mannose | + | 38 | Xylitol | - |
| 14 | L-Sorbose | - | 39 | Gentiobiose | + |
| 15 | L-Rhamnose | - | 40 | D-Turanose | - |
| 16 | Dulcitol | - | 41 | D-Lyxose | - |
| 17 | Inositol | - | 42 | D-Tagatose | + |
| 18 | D-Mannitol | - | 43 | D-Fucose | - |
| 19 | D-Sorbitol | - | 44 | L-Fucose | - |
| 20 | Méthyl-αD-Mannopyranoside | - | 45 | D-Arabitol | - |
| 21 | Méthyl-αD-Glucopyranoside | - | 46 | L-Arabitol | - |
| 22 | N-AcéthylGlucosamine | + | 47 | Potassium Gluconate | - |
| 23 | Amygdaline | + | 48 | Potassium 2-Cétogluconate | - |
| 24 | Arbutine | + | 49 | Potassium 5-Cétogluconate | - |
| 25 | Esculine citrate de fer | + | | | |

Il est à noter que l'identification biochimique des bacillus à partir du profil API 50CHB n'est pas fiable.

Par suite, l'identification de ces bactéries a été complétée par la caractérisation de leur génome (identification par séquençage du gène codant pour l'ARN16S).

Dans le cas particulier de la souche NOL 01 cette analyse n'a pas permis de différencier les espèces subtilis ou licheniformis.

Toutefois, l'aspect des colonies ferait davantage penser à Bacillus licheniformis.

Cinq des six souches bactériennes pouvant être renfermées dans la composition conforme à l'invention ont par ailleurs fait l'objet de dépôts auprès de l'Institut Pasteur selon le traité de Budapest sous les numéros suivants :

| | |
|---|---|
| NOL 01 | CNCM I - 4606 |
| NOL 03 | CNCM I - 4607 |
| NOL 04 | CNCM I - 4608 |
| NOL 11 | CNCM I - 4609 |
| NOL 12 | CNCM I - 4610 |

L'efficacité de la composition qui fait l'objet de l'invention pour le traitement de la colibacillose ainsi que l'effet de synergie des souches particulières de bacillus et de bactéries lactiques ont été confirmés par des tests in vitro à partir de boites de Pétri ensemencées par E.coli et également par des tests in vivo réalisés dans des élevages aviaires.

### Exemple 1 : Essais in vitro.

Mise en évidence de la synergie d'un bacillus et d'une bactérie lactique vis-à-vis de la croissance d'un E.coli.

On a successivement mis en œuvre les étapes ci-dessous :
Jour 1 : Repiquage des souches de bacillus et de bactéries lactiques sur une gélose au sang. Incubation des boites 20-24 h à 37°C.
Jour 2 : Préparation des boites de Pétri ∅ 90 mm.
   - couler 17 mL de gélose PCA en surfusion dans une boite de Pétri et laisser refroidir
   - retirer la gélose PCA sur une largeur de 4 mm (à l'aide de l'embout d'une pipette Pasteur) et une longueur d'½ strie centrale. La remplacer par de la gélose MRS en surfusion et laisser refroidir.
   Premier ensemencement des boites de Petri :
   - ensemencement d'une bactérie lactique sur la ½ strie centrale de gélose MRS
   - ensemencement d'un bacillus sur le prolongement de la ½ strie de la bactérie lactique
   - incubation 20-24 h à 37°C.
   Repiquer les souches d'E.coli sur une gélose Drigalski et incuber 20-24 h à 37°C.
Jour 3 : Préparation de la suspension d'E.coli :
   - à partir des colonies d'E.coli sur la gélose Drigalski, réaliser une suspension de Mac Farland 0.5 dans de l'eau physiologique
   - diluer 230 µL de cette suspension dans 4.2 mL d'eau physiologique.
   Deuxième ensemencement des boites de Pétri :
   - à l'aide d'un écouvillon et à partir de la dilution d'E.coli, badigeonner les boites de chaque côté des stries du bacillus et de la bactérie lactique
   - incuber 48 h à 37°C.
Jour 5 : Mesure (en mm) de la largeur des zones d'inhibition de la croissance d'E.coli :
   - ZI lactique = zone d'inhibition totale créée par la bactérie lactique (principalement causée par son activité acidifiante qui modifie le milieu et non par la bactérie elle-même)
   - ZI bacillus = zone d'inhibition totale créée par le bacillus
   - ZI synergie = zone d'inhibition totale créée par l'association du bacillus et de la bactérie lactique

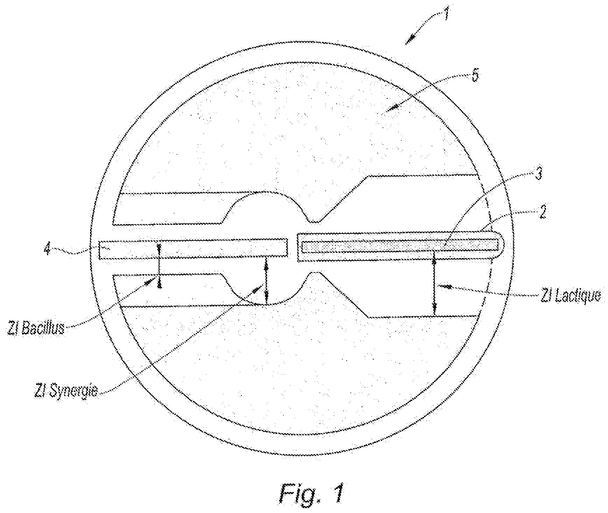

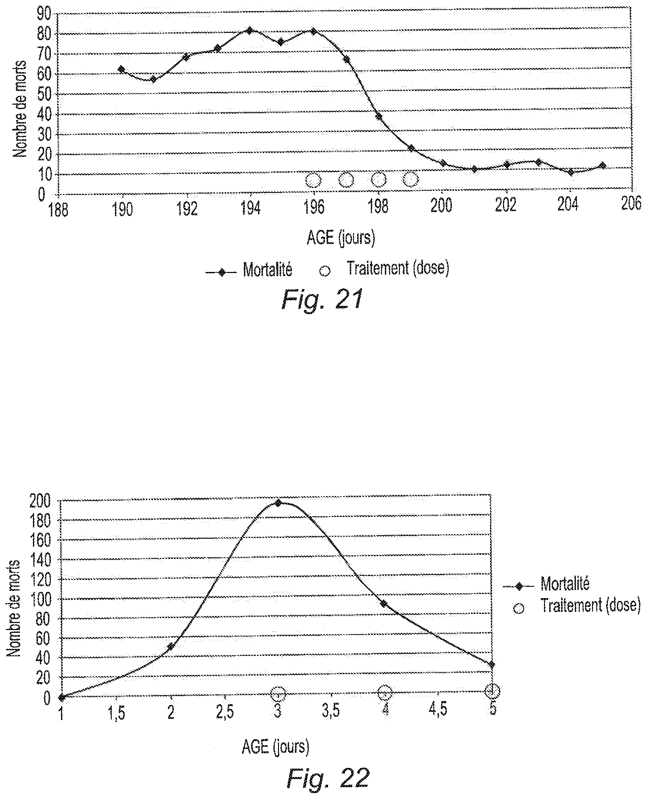

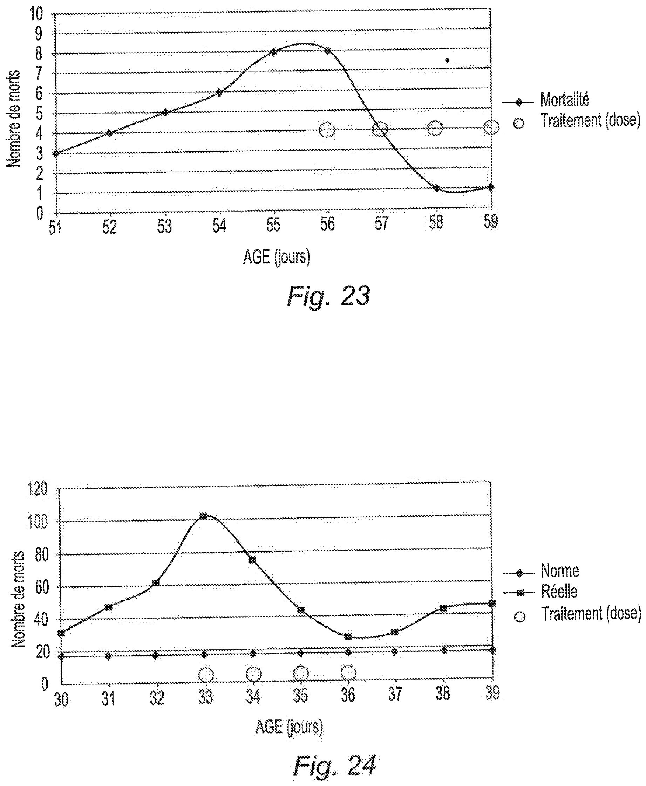

Une boite de Pétri 1 préparée de la manière susmentionnée est représentée schématiquement sur la figure 1.

La référence 2 correspond à la bande de gélose MRS sur laquelle est ensemencée une strie 3 d'une bactérie lactique.

La référence 4 correspond à une strie de bacillus ensemencée sur la gélose PCA dans le prolongement de la strie de la bactérie lactique.

La partie en grisé référencée 5 correspond à la zone de croissance de E-coli, alors que les zones blanches de part et d'autre des stries 3, 4 correspondent aux zones d'inhibition totale de cette croissance.

Les points de mesure respectifs de la largeur des zones d'inhibition de la croissance de E-coli : ZI lactique, ZI synergie et ZI bacillus sont représentées par des doubles flèches.

Cette figure montre clairement l'effet de synergie entre un bacillus et une bactérie lactique.

L'inhibition de la croissance de deux souches A et B de E.coli du fait de la synergie entre les différentes souches de bacillus et de bactéries lactiques peut être visualisée sur le tableau ci-dessous.

| INOCULATION | | | ZONE INHIBITION (mm) | | |
|---|---|---|---|---|---|
| bacillus | lactiques | E. coli | *lactique (acidité)* | bacillus | SYNERGIE |
| NOL 01 | NOL 11 | A | *1* | 0 | 3 |
| | | B | *1* | 0 | 4 |
| NOL 01 | NOL 12 | A | *13* | 2 | 3 |
| | | B | *14* | 0 | 3 |
| NOL 02 | NOL 11 | A | *1* | 2 | 0 |
| | | B | *1* | 2 | 0 |
| NOL 02 | NOL 12 | A | *10* | 3 | 0 |
| | | B | *12* | 3 | 0 |
| NOL 03 | NOL 11 | A | *1* | 0 | 5 |
| | | B | *2* | 0 | 6 |
| NOL 03 | NOL 12 | A | *12* | 0 | 5 |
| | | B | *15* | 0 | 5 |
| NOL 04 | NOL 12 | A | *10* | 0 | 3 |
| | | B | *14* | 0 | 5 |

Ces résultats montrent que les zones d'inhibition totale des bacillus sur la croissance de E.coli sont faibles et différentes suivant les souches.

En synergie avec une bactérie lactique, ces zones d'inhibition totale augmentent de façon nette dans le cas des bacillus NOL 01, NOL 03 et NOL 04 qui sont très peu actifs individuellement.

Cette synergie entre un bacillus et une bactérie lactique est en particulier prononcée dans le cas du bacillus NOL 03.

Au contraire des trois autres souches, le bacillus NOL 02 perd son activité inhibitrice vis-à-vis de la croissance des E.coli.

### Exemples 2 : Essais in vivo

Dans le cadre de ces essais on a préalablement préparé des flacons renfermant les préparations suivantes :
Préparation 1 = flacon de 20 ml
   mélange de 4 bacillus NOL 01, NOL 02, NOL 03, NOL 04 (sous la forme végétative)
   concentration de 10⁵ à 10⁸ ufc/ml
   conservation entre - 80°C et -21°C
Préparation 2 = flacon de 20 ml
   mélange de 2 bactéries lactiques (lactococcus + pediococcus) concentration de à 10¹⁰ ufc/ml
   conservation entre - 80°C et -21°C
Préparation 3 = flacon de 20 ml
   1 bacillus (NOL 04) (sous la forme végétative) concentration de 10⁵ à 10⁸ ufc/ml (formes revivifiables) conservation entre - 80°C et -21°C
on a ensuite testé sur le terrain les mélanges suivants : dose de mélange 1 : 1 flacon de préparation 1 + 1 flacon de préparation 2 dose de mélange 2 : 1 flacon de préparation 1 + 1 flacon de préparation 2

### Exemple 2.1 : Essai avec traitement par le mélange 1 sur des poules pondeuses :

Elevage : bâtiment de 56 000 poules
Examen laboratoire à 28 semaines : LA17656
Bactériologie : isolement d'un E.coli de sérotypie O78K80
Traitements : 5 doses par jour pendant 4 jours en eau de boisson sur la batterie n° 5
   5 doses par jour pendant 4 jours en nébulisation sur l'ensemble du bâtiment
   (5 doses pour 12 000 poules (batterie n° 5) → 25 tonnes soit 2 doses par 10 tonnes)
L'évolution de la mortalité est représentée sur le tableau ci-dessous ainsi que sur la figure 2.1

| AGE (jours) | mortalité | Traitement (dose) |
|---|---|---|
| 190 | 62 | |
| 191 | 57 | |
| 192 | 68 | |
| 193 | 72 | |
| 194 | 81 | |
| 195 | 75 | |
| 196 | 80 | 5 |
| 197 | 66 | 5 |
| 198 | 37 | 5 |
| 199 | 21 | 5 |
| 200 | 13 | |
| 201 | 10 | |
| 202 | 12 | |
| 203 | 13 | |
| 204 | 8 | |
| 205 | 11 | |

### Exemple 2.2 : Essai avec traitement par le mélange 1 sur des poulets de chair standard :

Elevage : bâtiment de 22 500 poulets
Examen laboratoire à 3 jours : LA17656
Autopsie : Omphalite
Bactériologie : isolement d'un E.coli de sérotypie O78K80
Traitements du jour 1 au jour 2 : enrofloxacine → échec du traitement expliqué par le résultat de l'examen effectué à J3 (l'E.coli O78K80 est résistant à l'enrofloxacine)
Suite à l'examen : 2 doses de mélange 1 par jour pendant 3 jours en eau de boisson (2 doses pour 22 500 poulets à 100 g → 2,2 tonnes soit 1 dose par tonne).
L'évolution de la mortalité du jour 1 au jour 5 est représentée sur le tableau ci-dessous ainsi que sur la figure 2.2

| AGE (jours) | mortalité | Traitement (dose) |
|---|---|---|
| 1 | 0 | |
| 2 | 50 | |
| 3 | 195 | 2 |
| 4 | 93 | 2 |
| 5 | 30 | 2 |

### Exemple 2.3 : Essai avec traitement par le mélange 2 sur des dindes de chair standards :

Elevage : bâtiment de 9 000 dindes
Examen laboratoire à 56 jours : LA19492
Autopsie : Lésions de colibacillose respiratoire
Bactériologie : isolement d'un E.coli de sérotypie (O78K80 ; O2 ; O1) négative
Traitement : 4 doses de mélange 2 par jour pendant 4 jours en eau de boisson (4 doses pour 9 000 dindes à 3,9 kg → 35 tonnes soit 1 dose pour 10 tonnes).
L'évolution de la mortalité entre 51 et 59 jours est représentée sur le tableau ci-dessous ainsi que sur la figure 2.3

| AGE (jours) | mortalité | Traitement (dose) |
|---|---|---|
| 51 | 3 | |
| 52 | 4 | |
| 53 | 5 | |
| 54 | 6 | |
| 55 | 8 | |
| 56 | 8 | 4 |
| 57 | 4 | 4 |
| 58 | 1 | 4 |
| 59 | 1 | 4 |

### Exemple 2.4 : Essai avec traitement par le mélange 1 sur des poulets de chair standard :

Elevage : bâtiment de 20 000 poulets
Examen laboratoire à 32 jours : LA18574
Autopsie : Péricardite/Arthrite/Nécrose de la tête fémorale
Bactériologie : isolement d'un E.coli de sérotypie (O78K80 ; O2 ; O1) négative
Traitement : 4 doses de mélange 1 par jour pendant 4 jours (4 doses pour 20 000 poulets à 1,4 kg → 28 tonnes soit 1,5 dose pour 10 tonnes). L'évolution de la mortalité entre 30 et 39 jours est représentée sur le tableau ci-dessous ainsi que sur la figure 2.4
Mortalité norme bien être : 1% + 0,06 % x N (N=nombre de jours d'élevage) 3,4 % (avec N=40 jours d'élevage)

Soit une mortalité totale de 680 morts (20 000 poulets x 3,4 %)
Soit une mortalité quotidienne moyenne de 17 morts (680 morts/40 jours)

| AGE (jours) | mortalité | | Traitement (dose) |
|---|---|---|---|
| | Norme (moyenne) | réelle | |
| 30 | 17 | 31 | |
| 31 | 17 | 47 | |
| 32 | 17 | 61 | |
| 33 | 17 | 101 | 4 |
| 34 | 17 | 74 | 4 |
| 35 | 17 | 43 | 4 |
| 36 | 17 | 27 | 4 |
| 37 | 17 | 29 | |
| 38 | 17 | 43 | |
| 38 | 17 | 45 | |

Ces résultats sont clairement de nature à prouver l'efficacité de la composition qui fait l'objet de l'invention pour le traitement de la colibacillose.

## Revendications

1. Composition bactérienne pour son utilisation dans le cadre du traitement de la colibacillose dans les élevages d'animaux en particulier les élevages aviaires, **caractérisée en ce qu'**elle renferme
- d'une part au moins 10⁵ ufc/ml d'au moins l'une des souches suivantes de Bacillus subtilis: NOL01 et NOL03,
- et d'autre part au moins 10⁵ ufc/ml d'au moins la bactérie lactique Lactococcus lactis spp lactis 1 souche NOL11,
où lesdites souches NOL01, NOL03 et NOL11 sont les suivantes :
∘ la souche NOL 01 est la souche déposée à l'Institut Pasteur sous le numéro CNCM I - 4606,
∘ la souche NOL 03 est la souche déposée à l'Institut Pasteur sous le numéro CNCM I - 4607, et
∘ la souche NOL 11 est la souche déposée à l'Institut Pasteur sous le numéro CNCM I - 4609.

2. Composition bactérienne pour son utilisation selon la revendication 1, **caractérisée en ce qu'**elle renferme entre 10⁵ et ufc/ml de bacillus et entre et 10¹⁰ ufc/ml de bactéries lactiques.

3. Composition pour son utilisation selon la revendication 1 ou 2, où lesdits bacillus et lesdites bactéries lactiques, sont sous forme sporulées et/ou végétatives.

4. Composition pour son utilisation selon l'une quelconque des revendications 1 à 3, **caractérisée en ce qu'**elle comprend l'une quelconque des compositions suivantes :
- la souche NOL 01 et la souche NOL 11, et
- la souche NOL 03 et la souche NOL 11.

5. Eau de boisson pour animaux d'élevage, en particulier pour volailles, **caractérisée en ce qu'**elle renferme la composition bactérienne conforme à l'une quelconque des revendications 1 à 4.

6. Composition bactérienne **caractérisée en ce qu'**elle renferme
- d'une part au moins 10⁵ ufc/ml d'au moins l'une des souches suivantes de Bacillus subtilis: NOL01 et NOL03,
- et d'autre part au moins 10⁵ ufc/ml d'au moins la bactérie lactique Lactococcus lactis spp lactis 1 souche NOL11,
où lesdites souches NOL01, NOL03 et NOL11 sont les suivantes :
∘ la souche NOL 01 est la souche déposée à l'Institut Pasteur sous le numéro CNCM I - 4606,
∘ la souche NOL 03 est la souche déposée à l'Institut Pasteur sous le numéro CNCM I - 4607, et
∘ la souche NOL 11 est la souche déposée à l'Institut Pasteur sous le numéro CNCM I - 4609.

7. Composition bactérienne selon la revendication 6, **caractérisée en ce qu'**elle renferme entre 10⁵ et ufc/ml de bacillus et entre 10⁹ et 10¹⁰ ufc/ml de bactéries lactiques.

8. Composition selon la revendication 6 ou 7, où lesdits bacillus et lesdites bactéries lactiques, sont sous forme sporulées et/ou végétatives.

9. Composition selon l'une quelconque des revendications 6 à 8, **caractérisée en ce qu'**elle comprend l'une quelconque des compositions suivantes :
- la souche NOL 01 et la souche NOL 11, et
- la souche NOL 03 et la souche NOL 11.

## Patentansprüche

1. Bakterienzusammensetzung zur Verwendung bei der Behandlung von Colibacillose bei Nutztieren, insbesondere in Geflügelzuchtbetrieben, **dadurch gekennzeichnet, dass** sie enthält
- einerseits mindestens 10⁵ KBE/ml von mindestens einem der folgenden Bacillus-subtilis-Stämme: NOL01 und NOL03,
- und andererseits mindestens 10⁵ KBE/ml des Milchsäurebakteriums Lactococcus lactis spp lactis 1, Stamm NOL11,
wobei die Stämme NOL01, NOL03 und NOL11 folgende sind:
∘ der Stamm NOL 01, der am Institut Pasteur unter der Nummer CNCM I-4606 hinterlegte Stamm ist,
∘ der Stamm NOL 03, der am Institut Pasteur unter der Nummer CNCM I-4607 hinterlegte Stamm ist, und
∘ der Stamm NOL 11, der am Institut Pasteur unter der Nummer CNCM I-4609 hinterlegte Stamm ist.

2. Bakterienzusammensetzung zur Verwendung nach Anspruch 1, **dadurch gekennzeichnet, dass** sie zwischen 10⁵ und 10⁹ KBE/ml Bacillus und zwischen 10⁹ und 10¹⁰ KBE/ml Milchsäurebakterien enthält.

3. Zusammensetzung zur Verwendung nach Anspruch 1 oder 2, wobei die Bacilli und die Milchsäurebakterien in sporenbildender und/oder vegetativer Form vorliegen.

4. Zusammensetzung zu ihrer Verwendung nach einem der Ansprüche 1 bis 3, **dadurch gekennzeichnet, dass** sie eine der folgenden Zusammensetzungen umfasst:
- Stamm NOL 01 und Stamm NOL 11 und
- Stamm NOL 03 und Stamm NOL 11.

5. Trinkwasser für Nutztiere, insbesondere Geflügel, **dadurch gekennzeichnet, dass** es die Bakterienzusammensetzung nach einem der Ansprüche 1 bis 4 enthält.

6. Bakterienzusammensetzung, **dadurch gekennzeichnet, dass** sie enthält
- einerseits mindestens 10⁵ KBE/ml von mindestens einem der folgenden Bacillus-subtilis-Stämme: NOL01 und NOL03,
- und andererseits mindestens 10⁵ KBE/ml des Milchsäurebakteriums Lactococcus lactis spp lactis 1, Stamm NOL11,
wobei die Stämme NOL01, NOL03 und NOL11 folgende sind:
∘ der Stamm NOL 01, der am Institut Pasteur unter der Nummer CNCM I-4606 hinterlegte Stamm ist,
∘ der Stamm NOL 03, der am Institut Pasteur unter der Nummer CNCM I-4607 hinterlegte Stamm ist, und
∘ der Stamm NOL 11, der am Institut Pasteur unter der Nummer CNCM I-4609 hinterlegte Stamm ist.

7. Bakterienzusammensetzung nach Anspruch 6, **dadurch gekennzeichnet, dass** sie zwischen 10⁵ und 10⁹ KBE/ml Bacillus und zwischen 10⁹ und 10¹⁰ KBE/ml Milchsäurebakterien enthält.

8. Zusammensetzung nach Anspruch 6 oder 7, wobei die Bacilli und die Milchsäurebakterien in sporenbildender und/oder vegetativer Form vorliegen.

9. Zusammensetzung nach einem der Ansprüche 6 bis 8, **dadurch gekennzeichnet, dass** sie eine der folgenden Zusammensetzungen umfasst:
- Stamm NOL 01 und Stamm NOL 11 und
- Stamm NOL 03 und Stamm NOL 11.

## Claims

1. A bacterial composition for its use for the treatment of colibacillosis on animal farms, in particular poultry farms, **characterised in that** it comprises
- on the one hand at least 10⁵ cfu/ml of at least one of the following strains of Bacillus subtilis: NOL01 and NOL03,
- and on the other hand at least 10⁵ cfu/ml of at least the lactic acid bacterium Lactococcus lactis spp lactis 1 strain NOL11,
wherein said strains NOL01, NOL03 and NOL11 are the following ones:
- the strain NOL 01 is the strain deposited at the Institut Pasteur under the number CNCM I - 4606,
- the strain NOL 03 is the strain deposited at the Institut Pasteur under the number CNCM I - 4607, and
- the strain NOL 11 is the strain deposited at the Institut Pasteur under the number CNCM I - 4609.

2. The bacterial composition for its use according to claim 1, **characterised in that** it comprises between 10⁵ and 10⁹ cfu/ml of bacillus and between 10⁹ and 10¹⁰ cfu/ml of lactic acid bacteria.

3. The composition for its use according to claim 1 or 2, wherein said bacillus and said lactic acid bacteria are in sporulated and/or vegetative form.

4. The composition for its use according to anyone of claims 1 to 3, **characterised in that** it comprises anyone of the following compositions:
- the strain NOL 01 and the strain NOL 11, and
- the strain NOL 03 and the strain NOL 11.

5. A drinking water for farm animals, in particular for poultry, **characterised in that** it comprises a bacterial composition according to anyone of claims 1 to 4.

6. A bacterial composition **characterised in that** it comprises
- on the one hand at least 10⁵ cfu/ml of at least one of the following strains of Bacillus subtilis: NOL01 and NOL03,
- and on the other hand at least 10⁵ cfu/ml of at least the lactic acid bacterium Lactococcus lactis spp lactis 1 strain NOL11,
wherein said strains NOL01, NOL03 and NOL11 are the following ones:
- the strain NOL01 is the strain deposited at the Institut Pasteur under the number CNCM I - 4606,
- the strain NOL03 is the strain deposited at the Institut Pasteur under the number CNCM I - 4607, and
- the strain NOL11 is the strain deposited at the Institut Pasteur under the number CNCM I - 4609.

7. The bacterial composition according to claim 6, **characterised in that** it comprises between 10⁵ and 10⁹ cfu/ml of bacillus and between 10⁹ and 10¹⁰ cfu/ml of lactic acid bacteria.

8. The composition according to claim 6 or 7, wherein said bacillus and said lactic acid bacteria are in sporulated and/or vegetative form.

9. The composition according to anyone of claims 6 to 8, **characterised in that** it comprises anyone of the following compositions:
- the strain NOL 01 and the strain NOL 11, and
- the strain NOL 03 and the strain NOL 11.
